Europäisches Patentamt

**European Patent Office**

**Office européen des brevets**

(19)

(11) Veröffentlichungsnummer: **0 181 546**
A1

(12)

# EUROPÄISCHE PATENTANMELDUNG

(21) Anmeldenummer: 85113609.3

(22) Anmeldetag: 25.10.85

(51) Int. Cl.⁴: **C 07 D 209/88**

(30) Priorität: 06.11.84 DE 3440486

(43) Veröffentlichungstag der Anmeldung:
21.05.86 Patentblatt 86/21

(84) Benannte Vertragsstaaten:
CH DE FR IT LI NL

(71) Anmelder: HOECHST AKTIENGESELLSCHAFT
Postfach 80 03 20
D-6230 Frankfurt am Main 80(DE)

(72) Erfinder: Neeb, Rudolf, Dr.
Am Klingenrain 23
D-6050 Offenbach/Main(DE)

(72) Erfinder: Tronich, Wolfgang, Dr.
Adolf-Guckes-Weg 5
D-6239 Eppstein/Taunus(DE)

(72) Erfinder: Volk, Heinrich, Dr.
Am Weissen Stein 1
D-6368 Bad Vilbel(DE)

(54) Verfahren zur Herstellung von 2-Hydroxy-carbazol-1-carbonsäure.

(57) 2-Hydroxy-carbazol-1-carbonsäure wird aus 2-Hydroxy-carbazol-3-carbonsäure hergestellt, indem man das Alkalisalz der 2-Hydroxy-carbazol-3-carbonsäure bei einer Temperatur von oberhalb 150°C einem Kohlendioxid-Druck von größer als 0,5 bar aussetzt.

EP 0 181 546 A1

Verfahren zur Herstellung von 2-Hydroxy-carbazol-
1-carbonsäure

Die Erfindung liegt auf dem technischen Gebiet der Synthese
von Zwischenprodukten.

2-Hydroxy-carbazol-1-carbonsäure ist ein wichtiges Zwischenprodukt für die Herstellung von Azofarbstoffen; es
dient insbesondere als Vorprodukt für Kupplungskomponenten
zur Herstellung von Azofarbstoffen auf der Faser in der
Eisfarbentechnik. Es kann in bekannter Weise durch Carboxylierung mittels Kohlendioxid von 2-Hydroxy-carbazol hergestellt werden, wobei gemäß dem Stand der Technik die
Carboxylierung als Feststoffcarboxylierungsreaktion oder
aber auch in einem Lösemittel erfolgen kann.

Bei der bekannten Feststoffcarboxylierungsreaktion wird
2-Hydroxy-carbazol in Form des trockenen Kaliumsalzes mittels Kohlendioxid unter Druck von etwa 4 bis 5 bar bei
Temperaturen bis zu 260°C umgesetzt (s. FIAT Final Report
No. 1313, Seiten 364-367). Bei dieser Carboxylierungsreaktion entsteht die isomere 2-Hydroxy-carbazol-3-carbonsäure
als unerwünschtes Nebenprodukt, die zur Weiterverarbeitung
bzw. Weiterverwendung der 2-Hydroxy-carbazol-1-carbonsäure
abgetrennt werden muß (in FIAT loc. cit. wird dem Hauptprodukt eine falsche Struktur, nämlich die des Nebenproduktes, zugeordnet; vgl. hierzu J. Chem. Soc. (C), 1969,
1518, und Beilsteins Handbuch der Organischen Chemie,
Band 22, Ergänzungswerk E III/IV, Seite 2360). Dieses Verfahren der Feststoffcarboxylierung hat den Nachteil, daß
das isomere Nebenprodukt, das noch bestimmte Anteile an
dem Hauptprodukt enthält, für die Gesamtausbeute einen
Verlust darstellt. Um es einer Weiterverwendung zuführen
zu können, muß es zunächst aufgearbeitet werden. Dies
geschieht durch Decarboxylierung der Hydroxycarbazolcarbonsäuren zum 2-Hydroxy-carbazol und dessen Abtrennung und Reinigung durch Sublimation, was insgesamt eine aufwendige technische Verfahrensweise darstellt.

Die bekannte Herstellung von 2-Hydroxy-carbazol-1-carbonsäure durch Carboxylierung des Kaliumsalzes von 2-Hydroxy-carbazol in Lösemitteln, wie beispielsweise in Wasser (Beispiel 2 der deutschen Patentschrift 512 234 - abgedruckt in Friedländer, Fortschritte der Teerfarbenfabrikation, Band 17, S. 734 + 735 (1932)) oder in organischen Lösemitteln, wie niederen aliphatischen Alkoholen, o-Dichlorbenzol und 2-Chlor-naphthalin (s. deutsche Offenlegungsschrift 33 13 906 und die dort als Stand der Technik zitierte Literatur) ist ebenfalls nicht frei von Nachteilen. Trotz eines gewissen Vorteiles, den einige dieser Verfahrensweisen hinsichtlich der Selektivität der Reaktion besitzen, haftet ihnen der Mangel an, daß das Carboxylierungsprodukt entweder durch extraktive Methoden aus dem Reaktionsmedium isoliert oder aber bei erhöhter Temperatur durch Filtration aus dem Reaktionsmedium gewonnen werden muß; dies bedingt jedoch aufwendige und geschlossene Filtrationsgeräte. Zudem muß das Lösemittel, auch dann, wenn es ohne Zwischenreinigung mehrmals einem Reaktionsansatz zugeführt werden kann, schließlich doch in einem zusätzlichen Arbeitsgang regeneriert oder vernichtet werden.

Es bestand deshalb weiterhin aus technischer Sicht der Wunsch, anstelle der aufwendigeren Verfahrensweisen in Lösemitteln die Verfahrensweise der Feststoffcarboxylierung zu verbessern.

Es wurde nun gefunden, daß eine aufwendige Aufarbeitung des isomeren Nebenproduktes bei der Feststoffcarboxylierung mit separater Decarboxylierung zum 2-Hydroxy-carbazol und dessen Isolierung durch Sublimation nicht mehr erforderlich ist, wenn man 2-Hydroxy-carbazol-3-carbonsäure,

- 3 -                                    0181546

die gegebenenfalls noch isomere 2-Hydroxy-carbazol-carbonsäuren und 2-Hydroxy-carbazol enthalten kann, in Form
ihrer Alkalisalze bei erhöhter Temperatur mit Kohlendioxid
unter Druck gemäß der Feststoffcarboxylierungsreaktion
umsetzt.

Die vorliegende Erfindung betrifft somit ein Verfahren zur
Herstellung von 2-Hydroxy-carbazol-1-carbonsäure bzw. dessen Alkalisalze durch Erhitzen eines trockenen Alkalisalzes, wie Natrium- oder Kaliumsalzes, bevorzugt des Kaliumsalzes, der 2-Hydroxy-carbazol-3-carbonsäure ohne Verwendung eines Lösemittels in Gegenwart von gasförmigem Kohlendioxid unter Anwendung von geringem bis starkem Reaktionsdruck.

Die lösemittelfreie Umsetzung des Alkalisalzes der
2-Hydroxy-carbazol-3-carbonsäure zum Alkalisalz der
2-Hydroxy-carbazol-1-carbonsäure kann analog den bekannten
Verfahrensweisen der Carboxylierung mittels Kohlendioxid
von 2-Hydroxy-carbazol erfolgen. So kann der Kohlendioxid-
Druck zwischen 0,5 bis 100 bar gewählt werden, vorzugsweise arbeitet man zwischen 1 und 10 bar und insbesondere zwischen 4 und 8 bar. Die Reaktionstemperatur kann zwischen
150 und 300°C liegen; vorzugsweise erfolgt die Umsetzung
bei einer Temperatur zwischen 180 und 280°C. Die Reaktionsgeschwindigkeit der erfindungsgemäßen Umsetzung richtet
sich natürlich nach den gewählten Bedingungen, wie Höhe des
Reaktionsdruckes und Höhe der Reaktionstempertur; demgemäß
beläuft sich die Reaktionszeit über zwei bis mehrere Stunden. Bei einer bevorzugten Verfahrensweise mit einem
Kohlendioxid-Druck von 1 bis 10 bar und einer Reaktionstemperatur von 180 bis 280°C beträgt die Reaktionszeit etwa
7 bis 20 Stunden.

Das erfindungsgemäße Verfahren hat den Vorteil, daß insbesondere das bei der üblichen Feststoffcarboxylierung von 2-Hydroxy-carbazol erhältliche Ausschußprodukt, wie die 2-Hydroxy-carbazol-3-carbonsäure bzw. deren Alkalisalz direkt nach der Abtrennung der gewünschten 2-Hydroxy-carbazol-1-carbonsäure ohne weitere Aufarbeitung der erfindungsgemäßen Carboxylierungsreaktion zur Wiederverwendung zugänglich ist. Das erfindungsgemäße Verfahren erlaubt es deshalb, das bei der herkömmlichen Carboxylierung von 2-Hydroxy-carbazol anfallende unerwünschte isomere Abfallprodukt direkt in das gewünschte Hauptprodukt umzuwandeln.

Eine besonders vorteilhafte Verfahrensweise des erfindungsgemäßen Verfahrens ist deshalb die Weiterverarbeitung des Ausschußproduktes der Carboxylierungsreaktion von 2-Hydroxy-carbazol, sei es, daß dieses Ausschußprodukt aus der üblichen Feststoffcarboxylierung von 2-Hydroxy-carbazol, sei es, daß es aus einem erfindungsgemäßen Ansatz selbst oder sei es gar, daß es aus einer Carboxylierungsreaktion von 2-Hydroxy-carbazol in einem Lösemittel herrührt, zur gewünschten 2-Hydroxy-carbazol-1-carbonsäure bzw. dessen Alkalisalz. Dieses isolierte Ausschußprodukt, das die 2-Hydroxy-carbazol-3-carbonsäure oder gegebenenfalls andere 2-Hydroxy-carbazol-carbonsäuren, einschließlich der nicht vollständig abtrennbaren 2-Hydroxy-carbazol-1-carbonsäure, enthält und das beispielsweise bei der Aufarbeitung des Carboxylierungsansatzes nach Abtrennung und Isolierung des 2-Hydroxy-carbazol-1-carbonsäure-Alkalisalzes durch Ansäuern des Filtrates gefällt und durch Absaugen gewonnen wird, kann zunächst mit der äquimolaren Menge an einem Alkalihydroxid, vorzugsweise Kaliumhydroxid, umgesetzt werden, das anschließend vollständig entwässert wird.

Der trockene Rückstand wird sodann unter den genannten Carboxylierungsbedingungen überwiegend in 2-Hydroxy-carbazol-1-carbonsäure (in Form deren Alkalisalze), das noch einen geringen Anteil an 2-Hydroxy-carbazol-3-carbonsäure (in Form deren Alkalisalze) enthält, umgewandelt.

Dieses Endprodukt kann sodann gemäß einer Verfahrensweise des Standes der Technik, wie beispielsweise in FIAT loc. cit. beschrieben, aufgearbeitet werden; nach Abtrennung der gewünschten 2-Hydroxy-carbazol-1-carbonsäure wird der Rückstand aus dem Filtrat - nach Zusatz von Alkalihydroxid - wie oben angegeben wiederum einer erneuten Umsetzung gemäß der erfindungsgemäßen Verfahrensweise zugeführt. Auf diese Weise gelingt es, die 2-Hydroxy-carbazol-3-carbonsäure (gegebenenfalls unter Zusatz von weiterer 2-Hydroxy-carbazol-3-carbonsäure aus anderen Reaktionsansätzen oder auch von 2-Hydroxy-carbazol) praktisch vollständig in 2-Hydroxy-carbazol-1-carbonsäure überzuführen.

Im einzelnen kann das erfindungsgemäße Verfahren wie folgt durchgeführt werden: Das aus der Feststoffcarboxylierungsreaktion gemäß einer bekannten Verfahrensweise oder gemäß dem erfindungsgemäßen Verfahren erhältliche Produkt wird in Wasser gelöst und nach einer Klärfiltration mit einem Alkalichlorid versetzt, wobei die Hauptmenge der gebildeten 2-Hydroxy-carbazol-1-carbonsäure als Alkalisalz abgeschieden und isoliert wird. Das in der Mutterlauge (Filtrat) verbleibende Gemisch der Alkalisalze aus 2-Hydroxy-carbazol-3-carbonsäure und restlicher 2-Hydroxy-carbazol-1-carbonsäure wird mittels einer Mineralsäure, wie Salzsäure oder Schwefelsäure, ausgefällt und isoliert, beispielsweise durch Filtration, und als Ausgangsmaterial unter Zusatz der äquimolaren Menge eines festen Alkalihydroxids, wie Kaliumhydroxid, in das erfindungsgemäße Verfahren eingesetzt.

Die nachfolgenden Beispiele dienen zur Erläuterung der Erfindung. Die Teile sind Gewichtsteile und die Prozentangaben beziehen sich auf Gewichtsprozente.

Beispiel 1

133 Teile (oder 0,5 Mol) trockenes Kaliumsalz der 2-Hydroxy-carbazol-3-carbonsäure werden in einer üblichen Carboxylierungsapparatur auf 260°C erhitzt und bei dieser Temperatur und bei einem Kohlendioxid-Druck von 5,5 bar etwa 10 Stunden unter Rühren gehalten. Das aus dem Ansatz gewonnene Reaktionsprodukt wird wie folgt aufgearbeitet: Es wird in 1400 Teilen Wasser gelöst, die Lösung mittels Salzsäure auf einen pH von 6,5 gestellt, in üblicher Weise, beispielsweise durch Filtration nach Zusatz von Aktivkohle oder Kieselgur, geklärt und die 2-Hydroxy-carbazol-1-carbonsäure durch Zugabe von 72 Teilen Natriumchlorid als deren Natriumsalz ausgesalzen, abfiltriert und bei 60°C getrocknet. Man erhält etwa 104 Teile eines Produktes, das neben 9 Teilen Natriumchlorid 92,1 Teile 100%iges 2-Hydroxy-carbazol-1-carbonsäure-Natriumsalz (entsprechend 84 Teilen reiner 2-Hydroxy-carbazol-1-carbonsäure) und 0,9 Teile 2-Hydroxy-carbazol-3-carbonsäure-Natriumsalz enthält. Dieses geringfügig durch die isomere 3-Carbonsäure verunreinigte 1-Carbonsäure-Produkt kann ohne weitere Aufarbeitung oder Reinigung beispielsweise direkt zur Herstellung von 2-Hydroxy-carbazol-1-carbonsäure-4-chloranilid (bekannt als (R)Naphtol AS-LB, C.I. Azoic Coupling Component 15) eingesetzt werden.

Aus dem Filtrat fallen beim Ansäuern auf einen pH-Wert von etwa 4 bis 2 mittels einer Mineralsäure, wie beispielsweise Salzsäure, noch 18 Teile eines Produktes aus, das zu etwa 65 % aus 2-Hydroxy-carbazol-3-carbonsäure und zu etwa 35 % aus 2-Hydroxy-carbazol-1-carbonsäure besteht. Dieses Produkt kann, nach dessen Überführung in das Alkalisalz, vorzugsweise Kaliumsalz, zusammen mit einem Alkalisalz der 2-Hydroxy-carbazol-3-carbonsäure anderer Provenienz und/ oder einem Alkalisalz des 2-Hydroxy-carbazols einem weiteren Ansatz des erfindungsgemäßen Verfahrens zugeführt werden.

Die Ausbeute an reiner 2-Hydroxy-carbazol-1-carbonsäure (84 Teile) entspricht 73,7 % d.Th., bezogen auf eingesetzte 2-Hydroxy-carbazol-3-carbonsäure.

Beispiel 2

Man verwendet ein Gemisch aus den trockenen Kaliumsalzen der 2-Hydroxy-carbazol-3-carbonsäure und der 2-Hydroxy-carbazol-1-carbonsäure, das beispielsweise bei der Aufarbeitung eines üblichen Carboxylierungsansatzes zur Herstellung von 2-Hydroxy-carbazol-1-carbonsäure durch Carboxylierung von 2-Hydroxy-carbazol-Kalium erhältlich ist. Geht man beispielsweise von 133 Teilen eines Gemisches aus 65 % 2-Hydroxy-carbazol-3-carbonsäure-Kalium und 35 % 2-Hydroxy-carbazol-1-carbonsäure-Kalium aus und setzt dies in erfindungsgemäßer Weise, wie bspw. im Beispiel 1 beschrieben, bei 260°C unter einem Kohlendioxid-Druck von 5,5 bar etwa 10 Stunden um und arbeitet es gemäß der im Beispiel 1 angegebenen Verfahrensweise auf, so erhält man 93,2 Teile 100%iges 2-Hydroxy-carbazol-1-carbonsäure-Natrium (entsprechend 85 Teilen reiner 2-Hydroxy-carbazol-1-carbonsäure) und 0,9 Teile 2-Hydroxy-carbazol-3-carbonsäure-Natrium (neben 5,9 Teilen Natriumchlorid).

Beispiel 3

Ein Gemisch aus 73 Teilen 2-Hydroxy-carbazol, 23 Teilen 2-Hydroxy-carbazol-3-carbonsäure und 56 Teilen einer 50%igen wäßrigen Lösung von Kaliumhydroxid wird zunächst unter Stickstoffatmosphäre bei Normaldruck auf 260°C erhitzt, wobei der größte Teil des im Gemisch enthaltenen Wassers abdestilliert. Die Schlußtrocknung erfolgt bei 260°C unter stark reduziertem Druck.
Das Gemisch der trockenen Kaliumsalze wird sodann, wie im Beispiel 1 beschrieben, mit Kohlendioxid umgesetzt. Nach Aufarbeitung des Reaktionsproduktes gemäß Beispiel 1 werden 102 Teile eines Produktes erhalten, das aus 92,1 Teilen 100%igem 2-Hydroxy-carbazol-1-carbonsäure-Natrium,
0,9 Teilen 2-Hydroxy-carbazol-3-carbonsäure-Natrium und
9 Teilen Natriumchlorid zusammengesetzt ist.


Beispiel 4

Man verfährt gemäß der Verfahrensweise des Beispieles 3,
verwendet jedoch nicht entsprechend dem Beispiel 1 zum
Aussalzen des 2-Hydroxy-carbazol-1-carbonsäure-Salzes
Natriumchlorid, sondern 50 Teile Kaliumchlorid. Man erhält
110 Teile eines Produktes, das aus 95,8 Teilen 100 %igem
2-Hydroxy-carbazol-1-carbonsäure-Kalium (entsprechend
82 Teilen reiner 2-Hydroxy-carbazol-1-carbonsäure) und
1,2 Teilen 2-Hydroxy-carbazol-3-carbonsäure-Kalium (neben
13 Teilen Kaliumchlorid) besteht.


Beispiel 5

Ein Gemisch aus 92 Teilen 2-Hydroxy-carbazol, 18 Teilen
eines Gemisches aus 65 % 2-Hydroxy-carbazol-3-carbonsäure und 35 % 2-Hydroxy-carbazol-1-carbonsäure (das durch
Ansäuern der Mutterlauge aus einem üblichen Carboxylierungsansatz von 2-Hydroxy-carbazol-Kalium erhalten wurde)
und 65 Teilen einer 50 %igen wäßrigen Lösung von Kaliumhydroxid wird gemäß den Angaben des Beispieles 3 entwässert und sodann in erfindungsgemäßer Weise, wie beispielsweise im Beispiel 1 angegeben, mit Kohlendioxid umgesetzt.
Die Aufarbeitung des Umsetzungsproduktes erfolgt sodann
gemäß Beispiel 1. Es werden 114 Teile 100%iges 2-Hydroxy-
carbazol-1-carbonsäure-Natrium (entsprechend 104 Teilen
reiner 2-Hydroxy-carbazol-1-carbonsäure) erhalten, das
noch durch 1 Teil 2-Hydroxy-carbazol-3-carbonsäure-Natrium
und 5 Teile Natriumchlorid verunreinigt ist.


Die aus der 2-Hydroxy-carbazol-1-carbonsäure-Natriumsalz-
Kristallisation erhältliche Mutterlauge wird mit 78%iger
wäßriger Schwefelsäure auf einen pH-Wert von 4 angesäuert.
Es fällt ein Produkt aus, das abgesaugt und getrocknet wird. Ausbeute: 18 Teile eines Gemisches, das zu etwa 65%
aus 2-Hydroxy-carbazol-3-carbonsäure und zu etwa 35% aus
2-Hydroxy-carbazol-1-carbonsäure besteht. Dieses Gemisch
kann einem Folgeansatz zur Herstellung von 2-Hydroxy-
carbazol-1-carbonsäure zugeschlagen werden.

0181546

PATENTANSPRÜCHE:

1. Verfahren zur Herstellung von 2-Hydroxy-carbazol-1-carbonsäure, dadurch gekennzeichnet, daß man ein Alkalisalz der 2-Hydroxy-carbazol-3-carbonsäure einer Temperatur von oberhalb 150°C und einem Kohlendioxid-Druck von oberhalb 0,5 bar aussetzt.

2. Verfahren nach Anspruch 1, dadurch gekennzeichnet, daß das Alkalisalz der 2-Hydroxy-carbazol-3-carbonsäure dessen Kaliumsalz ist.

3. Verfahren nach Anspruch 1 oder 2, dadurch gekennzeichnet, daß man das Salz der 2-Hydroxy-carbazol-3-carbonsäure einer Temperatur von 150 bis 300°C aussetzt.

4. Verfahren nach Anspruch 1 oder 2, dadurch gekennzeichnet, daß man das Salz der 2-Hydroxy-carbazol-3-carbonsäure einer Temperatur von 180 bis 280°C aussetzt.

5. Verfahren nach einem der Ansprüche 1 bis 4, dadurch gekennzeichnet, daß man das Salz der 2-Hydroxy-carbazol-3-carbonsäure einem Kohlendioxid-Druck von 0,5 bis 100 bar aussetzt.

6. Verfahren nach einem der Ansprüche 1 bis 4, dadurch gekennzeichnet, daß man das Salz der 2-Hydroxy-carbazol-3-carbonsäure einem Kohlendioxid-Druck von 1 bis 10 bar aussetzt.

7. Verfahren nach einem der Ansprüche 1 bis 6, dadurch gekennzeichnet, daß man ein Gemisch der Alkalisalze, vorzugsweise Kaliumsalze, von 2-Hydroxy-carbazol-carbonsäuren einsetzt, das aus der Aufarbeitung eines Carboxylierungsansatzes von 2-Hydroxy-carbazol erhältlich ist.

8. Verfahren nach Anspruch 7, dadurch gekennzeichnet, daß dieses Ausgangsgemisch zusätzlich ein Alkalisalz, vorzugsweise Kaliumsalz, des 2-Hydroxy-carbazols enthält.

9. Verfahren nach einem der Ansprüche 1 bis 6, dadurch gekennzeichnet, daß man ein Gemisch der Alkalisalze, vorzugsweise Kaliumsalze des Aufarbeitungsproduktes mit den 2-Hydroxy-carbazol-3- und -1-carbonsäuren aus einem der Verfahren nach den Ansprüchen 1 bis 6 als Ausgangsprodukt einsetzt.

0181546
Nummer der Anmeldung

EUROPÄISCHER RECHERCHENBERICHT

EP 85 11 3609

Europäisches
Patentamt

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | HOUBEN-WEYL, "Methoden der organischen Chemie", Band VIII, Kapitel 4, Seiten 365-502, 4. Auflage, 1973, Georg Thieme Verlag; Stuttgart; H. HENECKA "Methoden zur Herstellung, Umwandlung und Decarboxylierung von Carbonsäuren" * Seite 372, Zeilen 21-31; Seite 374, Zeile 29 - Seite 375, Zeile 4; Seite 375, Zeilen 27-32, Seite 493, Zeilen 9-19, Fussnote 3; Seite 494, Zeilen 14-18 * | 1-9 | C 07 D 209/88 |
| Y | DE-C- 522 960 (I.G. FARBENINDUSTRIE AG) * Beispiele 3, 4 * | 1-9 | |
| Y | CHEMICAL ABSTRACTS, Band 72, Nr. 9, 3. März 1970, Seite 79, Spalte 1, Abstrakt Nr. 44997w, Columbus, Ohio, US; M.R.R. BHAGWANTH et al.: "Applications of NMR and mass spectroscopy to some problems concerning synthetic dyes. V. Kolbe-Schmitt reaction products from 2-hydroxycarbazole and the structure of Naphthol AS-LB", & INDIAN J. CHEM. 1969, 7(11), 1065-1069<br><br>--- -/- | 1-9 | RECHERCHIERTE SACHGEBIETE (Int Cl 4)<br><br>C 07 D 209/00 |

Der vorliegende Recherchenbericht wurde für alle Patentansprüche erstellt

| Recherchenort | Abschlußdatum der Recherche | Prüfer |
|---|---|---|
| BERLIN | 03-02-1986 | VAN AMSTERDAM L.J.P. |

| | Europäisches Patentamt | **EUROPÄISCHER RECHERCHENBERICHT** | | |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| A,D | DE-C- 512 234 (I.G. FARBENINDUSTRIE AG) * Beispiele 1, 3, 4 * | 1-9 | |
| A,D | BEILSTEINS HANDBUCH DER ORGANISCHEN CHEMIE, 4. Auflage, Band E III/IV 22, Teil 3, 1979, Springer-Verlag, Berlin * Seite 2360, 2-Hydroxy-carbazol-1-carbonsäure, Seite 2363, 2-Hydroxy-carbazol-3-carbonsäure * | 1-9 | |
| A | EP-A-0 110 239 (BAYER AG) * Ansprüche * & DE - A - 3 313 906 (Kat. D) | 1-9 | |

RECHERCHIERTE SACHGEBIETE (Int. Cl.4)

Der vorliegende Recherchenbericht wurde für alle Patentansprüche erstellt

| Recherchenort BERLIN | Abschlußdatum der Recherche 03-02-1986 | Prüfer VAN AMSTERDAM L.J.P. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82